Europäisches Patentamt

European Patent Office

Office européen des brevets

(11) Veröffentlichungsnummer: **0 520 273 A2**

## EUROPÄISCHE PATENTANMELDUNG

(21) Anmeldenummer: **92110039.2**

(22) Anmeldetag: **15.06.92**

(51) Int. Cl.⁵: **C07C 269/04**, C07C 263/04

(30) Priorität: **27.06.91 DE 4121211**

(43) Veröffentlichungstag der Anmeldung:
**30.12.92 Patentblatt 92/53**

(84) Benannte Vertragsstaaten:
**AT BE CH DE ES FR GB IT LI NL**

(71) Anmelder: **BAYER AG**

**W-5090 Leverkusen 1 Bayerwerk(DE)**

(72) Erfinder: **Heitkämper, Peter, Dr.**
**Fliederweg 14**
**W-4047 Dormagen 11(DE)**
Erfinder: **Schieb, Thomas, Dr.**
**Vierkotter Feld 1**
**W-5064 Rösrath(DE)**
Erfinder: **Wershofen, Stefan, Dr.**
**Nahestrasse 28**
**W-4050 Mönchengladbach(DE)**

(54) Verfahren zur Herstellung von Bis(ethoxycarbonyl-amino)toluolen und deren Verwendung zur Herstellung von Diisocyanatotoluolen.

(57) Ein Verfahren zur Herstellung von Bis-(ethoxycarbonylamino)toluolen durch Umsetzung von Diaminotoluolen mit Diethylcarbonat in Gegenwart eines Katalysators bei 100 bis 300°C unter gleichzeitiger oder anschließender destillativer Entfernung des gebildeten Alkohols, wobei man das Diethylcarbonat in einem mindestens 5-fach molaren überschuß bezogen auf die Aminogruppen des Diaminotoluols verwendet und die gebildeten Bis-(ethoxycarbonylamino)toluole nach Abkühlen des Reaktionsgemischs in Form der hierbei anfallenden Kristalle hoher Reinheit isoliert, sowie die Verwendung der so erhaltenen Bis(ethoxycarbonylamino)-toluole als Ausgangsmaterialien bei der Herstellung von Diisocyanatotoluolen.

EP 0 520 273 A2

Die Erfindung betrifft ein verbessertes Verfahren zur Herstellung von Bis(ethoxycarbonylamino)-toluolen ( = Toluylenbis(O-ethylurethanen)) durch Umsetzung von Diaminotoluolen mit Diethylcarbonat in Gegenwart eines Katalysators und Isolierung der gebildeten Bis(O-ethylurethane) in hoher Reinheit, sowie die Verwendung der Verfahrensprodukte als Ausgangsmaterialien zur Herstellung von Diisocyanatotoluolen.

Die Herstellung von Urethanen durch Umsetzung von Aminen mit organischen Carbonaten ist bekannt. Zur Aufarbeitung der Reaktionsgemische und Isolierung der reinen Produkte sind in der Literatur verschiedene Verfahrensweisen beschrieben.

Monourethane können z.B. durch Destillation gereinigt und isoliert werden (DE-OS 3 035 354, EP-A-O 323 514, EP-A-O 391 473). Diese Methode ist jedoch auf Toluylen-bis(O-alkylurethane) nicht anwendbar, da sich diese Urethane nicht unzersetzt destillieren lassen.

Eine andere Möglichkeit zur Reinigung der bei der Umsetzung von Aminen mit Carbonaten gebildeten Urethane ist das Umkristallisieren. Gemäß DE-OS 2 160 111 wird z.B. nach der Reaktion von Anilin mit Dimethylcarbonat als Nebenprodukt entstandener Diphenylharnstoff abfiltriert, das Filtrat eingeengt und der Rückstand aus Hexan umkristallisiert, um N-Phenyl-O-methylurethan zu gewinnen. Das Verfahrensprodukt enthält jedoch auch nach Umkristallisieren als Verunreinigung noch ca. 20 % N-alkylierte Aniline, die als Nebenprodukte bei der Reaktion angefallen sind.

2,4-Bis(ethoxycarbonylamino)toluol kann gemäß Beispiel 8 der DE-OS 3 035 354 nach der Titantetrabutylat-katalysierten Umsetzung von 2,4-Diaminotoluol mit überschüssigem Diethylcarbonat isoliert werden, indem der als Hauptprodukt gebildete Polyharnstoff abfiltriert wird, das Filtrat eingeengt und der Rückstand aus Toluol umkristallisiert wird. Nachteilig bei diesem Verfahren ist die geringe Ausbeute an Diurethan (26 % d.Th.) und die Bildung großer Mengen an Polyharnstoff, der in einem zusätzlichen Verfahrensschritt ins Diurethan übergeführt werden muß.

Aufgabe der vorliegenden Erfindung ist daher, ein Verfahren zur Herstellung von Toluylenbis(O-alkylurethanen) zur Verfügung zu stellen, das die Herstellung und Isolierung der Produkte in hoher Reinheit durch einfache und effiziente Aufarbeitung ermöglicht, ohne daß die oben beschriebenen Nachteile bekannter Verfahren auftreten.

Diese Aufgabe konnte am konkreten Beispiel der Bis-(ethoxycarbonylamino)toluole (Toluylenbis-(O-ethylurethane)) überraschenderweise dadurch gelöst werden, daß bei der Umsetzung das Diethylcarbonat in sehr hohem Überschuß zur Anwendung gelangt, was zur Folge hat, daß die Bildung von Nebenprodukten, insbesondere von vorzeitig auskristallisierenden Polyharnstoffen weitgehend unterbleibt, so daß das angestrebte Verfahrensprodukt in hoher Ausbeute auf einfache Weise in hoher Reinheit isoliert werden kann.

Gegenstand der Erfindung ist ein Verfahren zur Herstellung von Bis(ethoxycarbonylamino)toluolen durch Umsetzung von Diaminotoluolen mit Diethylcarbonat in Gegenwart eines Katalysators bei 100 bis 300°C unter gleichzeitiger oder anschließender destillativer Entfernung des gebildeten Alkohols, dadurch gekennzeichnet, daß man das Diethylcarbonat, bezogen auf die Aminogruppen des Diaminotoluols, in einem mindestens 5-fach molaren Überschuß verwendet und die gebildeten Bis-(ethoxycarbonylamino)toluole nach Abkühlen und gegebenenfalls nach teilweisem Einengen des Reaktionsgemischs ohne vorherige Abtrennung von Nebenprodukten in Form der hierbei anfallenden Kristalle hoher Reinheit isoliert.

Gegenstand der Erfindung ist auch die Verwendung der nach diesem Verfahren erhaltenen Bis-(ethoxycarbonylamino)toluole als Ausgangsmaterialien bei der Herstellung von Diisocyanatotoluolen durch thermische Spaltung der Bis(O-ethylurethane).

Für das erfindungsgemäße Verfahren können beliebige Diaminotoluole eingesetzt werden. Bevorzugte Ausgangsmaterialien sind jedoch 2,4-Diaminotoluol und seine technischen Gemische mit bis zu 40, vorzugsweise bis zu 35 Gew.-%, bezogen auf Gemisch, an 2,6-Diaminotoluol.

Als Katalysatoren eignen sich Metallverbindungen der 1. bis 5. Hauptgruppe und der 1. bis 8. Nebengruppe des Periodensystems der Elemente.

Vorzugsweise werden Metallverbindungen der 4. Hauptgruppe und der 2. Nebengruppe des Periodensystems als Katalysatoren eingesetzt.

Besonders bevorzugt werden Verbindungen des Zinns, des Zinks und des Bleis eingesetzt.

Beispiele für derartige Katalysatoren sind
- Lewis-Säuren wie z.B. Salze oder Verbindungen von Zink, Blei, Titan oder Zirkonium, wie z.B. Zink(II)chlorid, Zink(II)acetat und andere Zink(II)carboxylate, Blei(II)acetat und andere Blei(II)carboxylate, Titantetrabutylat oder Zirkoniumtetrapropylat,
- zinnorganische Verbindungen wie z.B. Dibutylzinnoxid oder Dibutylzinndilaurat,
- basische Verbindungen wie z.B. Alkali- oder Erdalkalihydroxide oder -alkoxide.

Bevorzugt sind solche Katalysatoren, die, wenn sie im Produkt verbleiben, eine nachfolgende Spaltung der bei der erfindungsgemäßen Umsetzung gebildeten Urethane in die entsprechenden Isocyanate und Alkohole nicht nachteilig beeinflussen. Hierzu gehören insbesondere die obengenannten besonders bevorzugten Katalysatoren.

Die Katalysatoren werden in Mengen von 0,01 bis 20 Mol-%, bevorzugt 0,05 bis 15 Mol-%, besonders bevorzugt 0,1 bis 10 Mol-%, bezogen auf eingesetztes Diamin, eingesetzt.

Die Reaktionstemperaturen liegen im Bereich von 100 bis 300°C, bevorzugt im Bereich von 120 bis 250°C. Das Verfahren kann bei Normaldruck oder erhöhtem Druck durchgeführt werden. Erhöhter Druck ist erforderlich, wenn die Reaktionstemperatur über dem Siedepunkt des Reaktionsgemischs bei Normaldruck liegt.

Es ist ein wesentlicher Punkt, daß das eingesetzte Diethylcarbonat, bezogen auf das Diamin, in hohem Überschuß zur Anwendung gelangt. Im allgemeinen wird das Diethylcarbonat, bezogen auf die Aminogruppen des Diamins in mindestens 5-fach, vorzugsweise mindestens 10-fach und besonders bevorzugt in 10- bis 30-fach molarem Überschuß eingesetzt.

Das im Überschuß vorliegende Diethylcarbonat hat die wichtige Funktion eines Lösungsmittels, dessen Anwesenheit offensichtlich für die Ausbeutemaximierung und Reindarstellung des Verfahrensprodukts durch einfaches Kristallisieren wesentlich ist. Der sich während der Umsetzung gemäß folgender Reaktionsgleichung

$$R\text{-}(NH_2)_n + n\ (R'O)_2CO \rightarrow R\text{-}(NHCOOR')_n + n\ R'OH$$

(die Reste R und R' stehen in dieser lediglich das Reaktionsprinzip erläuternden Gleichung für die indifferenten Reste der Reaktionspartner) bildende Alkohol wird entweder fortlaufend während der Reaktion oder zu einem geeigneten Zeitpunkt nach beendeter Umsetzung destillativ abgetrennt.

Die überraschende Beobachtung bestand darin, daß beim erfindungsgemäßen Verfahren im Gegensatz zum Verfahren der DE-OS 3 035 354 keine nennenswerte Mengen an beim Abkühlen des Reaktionsgemischs auskristallisierenden Nebenprodukten, insbesondere Polyharnstoffen entstehen, so daß die gebildeten Bis(O-ethylurethane) ohne weitere Reinigung nach Abkühlen des Reaktionsgemischs auf unterhalb 80°C, vorzugsweise unterhalb 50°C liegende Temperaturen und gegebenenfalls erfolgtem Einengen in hoher Reinheit (Reinheitsgrad mindestens 95, vorzugsweise mindestens 97 Gew.-%) kristallin anfallen und durch einfaches Filtrieren und gegebenenfalls nachfolgendes Waschen mit Diethylcarbonat isoliert werden können. Die so erhaltenen Verfahrensprodukte entsprechen bezüglich ihrer Isomerenverteilung weitgehend der Isomerenverteilung der eingesetzten Diaminotoluole. Bei den Verfahrensprodukten handelt es sich somit im wesentlichen um 2,4-Bis-(ethoxycarbonylamino)toluol oder um dessen Gemische mit bis zu 40, vorzugsweise bis zu 35 Gew.-%, bezogen auf Gemisch, an 2,6-Bis-(ethoxycarbonylamino)toluol bei einer Reinheit der genannten Produkte von mindestens 95 Gew.-%, vorzugsweise mindestens 97 Gew.-%.

Das nach dem Abtrennen der Verfahrensprodukte verbleibende Filtrat enthält neben Diurethanen im wesentlichen Aminourethane sowie andere Neben- und Zwischenprodukte, die sich durch erneute Umsetzung mit Dialkylcarbonaten in Diurethane überführen lassen, sowie gegebenenfalls geringe Mengen an N-alkylierten Verbindungen. Das Filtrat kann, gegebenenfalls nach Abtrennung von bei der Umsetzung gebildetem Alkohol und gegebenenfalls nach Abtrennung von einem Teil der N-alkylierten Nebenprodukte, erneut dem erfindungsgemäßen Verfahren zugeführt werden. Ebenso kann das gegebenenfalls durch Einengen des Reaktionsgemisches anfallende Destillat nach Abtrennung von bei der Umsetzung entstandenem Alkohol erneut dem erfindungsgemäßen Verfahren zugeführt werden.

Das erfindungsgemäße Verfahren kann kontinuierlich oder diskontinuierlich durchgeführt werden.

Die nach dem erfindungsgemäßen Verfahren hergestellten Urethane können ohne weitere Aufarbeitung zur Herstellung der entsprechenden Isocyanate verwendet werden. Dabei werden die Urethane durch thermische Spaltung in die entsprechenden Isocyanate und Alkohole überführt und die entstehenden Spaltprodukte nachfolgend getrennt.

Das erfindungsgemäße Verfahren soll anhand der nachfolgenden Beispiele näher erläutert werden. Alle Prozentangaben beziehen sich auf das Gewicht.

Beispiel 1

Ein Gemisch aus 48,8 g (0,4 Mol) 2,4-Diaminotoluol, 1890 g (16 Mol) Diethylcarbonat (entsprechend einem Molverhältnis von Aminogruppen zu Carbonat von 1:20) und 3,6 g einer Katalysatorlösung, bestehend aus einem Gemisch von Bleisalzen der 2-Ethylhexan- und Isononansäure, gelöst in Testbenzin (Blei-Gehalt der Lösung 24 %) wird in einem 3 l Autoklaven auf 200°C aufgeheizt. Nach 2 Stunden wird die Reaktion durch Abkühlen auf 25°C abgebrochen. Das Reaktionsgemisch wird im Wasserstrahlvakuum eingeengt. Der ausgefallene Feststoff wird abfiltriert, mit 50 ml Diethylcarbonat gewaschen und getrocknet. Man erhält 75,7 g 2,4-Bis(ethoxycarbonylamino)toluol (Reinheit 98 %, Schmelzpunkt 133°C), entsprechend einer Ausbeute von 71 % der Theorie. Durch Rückführung der Filtrate kann die Ausbeute an isoliertem 2,4-Bis(ethoxycarbonylamino)toluol auf ca. 95 % der Theorie gesteigert werden.

Beispiel 2

Ein Gemisch aus 48,8 g (0,4 Mol) Diaminotoluol (technisches Gemisch der 2,4- und 2,6-Isomeren im Gewichtsverhältnis 65:35), 1890 g (16 Mol) Diethylcarbonat und 3,6 g der in Beispiel 1 beschriebenen Katalysatorlösung wird in einem 3 l Autoklaven auf 200°C aufgeheizt. Nach 4 Stunden wird die Reaktion durch Abkühlen auf 25°C abgebrochen. Das Reaktionsgemisch wird im Wasserstrahlvakuum eingeengt. Der ausgefallene Feststoff wird abfiltriert, mit 80 ml Diethylcarbonat gewaschen und getrocknet. Man erhält 63,2 g 2,4-/2,6-Bis(ethoxycarbonylamino)toluol (Gehalt des 2,6-Isomeren 32 %), entsprechend einer Ausbeute von 59 % der Theorie. Durch Rückführung der Filtrate kann die Ausbeute an isoliertem 2,4-/2,6-Bis-(ethoxycarbonylamino)toluol auf über 90 % der Theorie gesteigert werden.

**Patentansprüche**

1. Verfahren zur Herstellung von Bis-(ethoxycarbonylamino)toluolen durch Umsetzung von Diaminotoluolen mit Diethylcarbonat in Gegenwart eines Katalysators bei 100 bis 300°C unter gleichzeitiger oder anschließender destillativer Entfernung des gebildeten Alkohols, dadurch gekennzeichnet, daß man das Diethylcarbonat, bezogen auf die Aminogruppen des Diaminotoluols in einem mindestens 5-fach molaren Überschuß verwendet und die gebildeten Bis(ethoxycarbonylamino)toluole nach Abkühlen und gegebenenfalls nach teilweisem Einengen des Reaktionsgemischs ohne vorherige Abtrennung von Nebenprodukten in Form der hierbei anfallenden Kristalle hoher Reinheit isoliert.

2. Verfahren gemäß Anspruch 1, dadurch gekennzeichnet, daß man als Diaminotoluol 2,4-Diaminotoluol oder dessen technische Gemische mit bis zu 40 Gew.-%, bezogen auf Gemisch, an 2,6-Diaminotoluol verwendet.

3. Verfahren gemäß Anspruch 1 und 2, dadurch gekennzeichnet, daß man als Katalysatoren Metallverbindungen von Metallen der 1. bis 5. Hauptgruppe oder der 1. bis 8. Nebengruppe des Periodensystems der Elemente verwendet.

4. Verfahren gemäß Anspruch 1 bis 3, dadurch gekennzeichnet, daß man als Katalysatoren Metallverbindungen der 4. Hauptgruppe oder der 2. Nebengruppe des Periodensystems der Elemente verwendet.

5. Verfahren gemäß Anspruch 1 bis 4, dadurch gekennzeichnet, daß man als Katalysatoren Verbindungen des Zinns, des Zinks oder des Bleis verwendet.

6. Verwendung der gemäß Ansprüchen 1 bis 5 erhaltenen Bis(ethoxycarbonylamino)toluole als Ausgangsmaterialien bei der Herstellung von Diisocyanatotoluolen durch thermische Spaltung der Bis(O-ethylurethane).